# EUROPEAN PATENT SPECIFICATION

(11) **EP 3 062 731 B1**
(45) Date of publication and mention of the grant of the patent: **17.04.2019**
(21) Application number: 14798947.9
(22) Date of filing: 26.09.2014
(51) Int. Cl.: A61C 8/00, A61C 8/02

(54) **DENTAL IMPLANT**
ZAHNIMPLANTAT
IMPLANT DENTAIRE

(30) Priority: 29.10.2013 IT MI20131796
(43) Date of publication of application: 07.09.2016
(73) Proprietor: SPIDERIMPLANT S.r.l., 61032 Fano (PS) (IT)
(72) Inventor: ZANNA Massimo, 61032 Fano (IT)
(74) Representative: Gregorj S.r.l.
(86) International application number: PCT/IB2014/064872
(87) International publication number: WO 2015/063625

(56) References cited:
- WO-A1-01/82822
- WO-A1-02/074181
- WO-A1-2008/127075
- WO-A1-2014/138135
- WO-A2-2011/010061
- US-A- 5 052 930
- US-A- 5 853 291
- US-A- 5 976 142
- US-A1- 2008 081 315
- US-A1- 2009 317 765
- US-A1- 2012 196 250

## Description

### Technical field of the invention

The present invention refers to a dental implant for rehabilitating patients affected by complete or partial edentulism, in other words the loss or absence of one or more teeth.

### Prior art

Dental implants to be generally applied in the mandibular or maxillary bone of patients who have lost or have experienced a damage to one or more teeth are known.

A type of known implant comprises a cylindrical or conical screw, normally made of titanium, to be screwed in a suitable threaded seat previously formed in the mandibular or maxillary bone of the patient. The screw has the function of forming an artificial root for an artificial tooth, which is applied, normally by a stump screwable in a suitable seat of the screw, to an end of the screw itself once the latter has been completely osteointegrated.

However, such type of dental implant has functional limits in case the implant is applied in a zone wherein the quantity of bone is scarce, particularly the cortical bone (representing the hard part of a bone, different from the spongy bone which is soft and yielding). In such a circumstance, the implant screw cannot have at its disposal a sufficient gripping surface and therefore it often gets loose, in other words, it goes out the seat formed in the bone, consequently the patient loses the implant.

In order to overcome the above mentioned shortcomings, different solutions have been proposed.

A first solution consists of the so-called osteoregeneration, in other words the use of techniques promoting the physiologic process of regrowing the bone. Illustratively, such techniques comprise the autologous graft, the implant of an artificial bone made of synthetic material, the osteoinduction. However, such techniques involve several risks and shortcomings, such as the possible rejection, besides high difficulties in executing the operation.

A second solution is represented by the so-called zygomatic implants, which exploits the zygomatic bone of the patient in order to install the dental implant. Such technique comprises the insertion of long posts extending from the insertion zone of the implant to the zygomatic zone. Therefore, the operation is very complex and invasive. Further, since the zygoma is near to the patient's eye, the operation should be performed accurately and precisely because, in case of a mistake, the sight could be badly compromised.

Lastly, a third solution consists of the so-called "short" implants, functionally analogous to the described implants, but using screws having a small axial size, expressly designed for being inserted in the cortical bone, even though it is present in small quantities. However, neither such implants are still totally reliable and it is not uncommon for a patient to lose them.

Further implants according to the prior art are described in documents US 5 052 930 A, WO 2011/010061 A2, US 2006/154205 A1, US 5 976 142 A, WO 02/074181, US 2008/081315 A1, US 2003/232308 A1, WO 02/45613.

### Summary of the invention

Therefore, an object of the present invention is to provide a dental implant which is reliable, in other words having a high retainability, and providing an almost complete osteointegration also in the presence of scarce bone amounts, and which is sufficiently simple to be installed.

Such object is met by a dental implant comprising a main body and secondary screws fixedly coupable to an auxiliary plate in order to form an endosseous implant realizing only one implantologic entity with the osteointegrated surface. Indeed, the Applicant has observed that the secondary screws coupled to the auxiliary plates of the implants described in the prior art documents, are simple compression screws, in other words suitable for compressing the auxiliary plate in the implant seat, which however are not interference fitted with the auxiliary plate and therefore do not form a body integral with the latter. The osteointegration ensured by this kind of solution is not satisfying because it does not involve the implant in its entirety and therefore also its reliability can be insufficient.

This and other objects are met by a dental implant according to claim 1.

### Brief description of the drawings

To better understand the invention and appreciate its advantages, in the following some exemplifying non limiting embodiments thereof will be described, with reference to the attached drawings, wherein:
Figure 1 is a perspective view of a dental implant according to a possible embodiment;
Figure 2 is an exploded perspective view of the dental implant according to a further possible embodiment;
Figures 3a-3c are a perspective view, a lateral partially cross-section view, and a plan view, respectively, of a main body of the implant in Figure 2;
Figures 4a and 4b are a lateral view and a cross-section view, respectively, of a reinforcing portion of an auxiliary plate of the dental implant in Figure 2;
Figure 5 illustrates auxiliary plates of the dental implant according to some possible alternative embodiments;
Figure 6 is a perspective view of an auxiliary plate of the dental implant according to a further possible embodiment of the invention;
Figure 7 is a schematic view of an implanted dental implant according to an alternative embodiment.

### Detailed description of the invention

With reference to the attached figures, reference 1 shows a dental implant. Generally the dental implant 1 is destined to be applied in the mandibular or maxillary bone of a patient who lost one or more teeth which therefore must be substituted.

The dental implant 1 comprises a main body 2, preferably having an elongated shape, for example a generally cylindrical or frustoconical, or partially cylindrical and partially conical shape, destined to be implanted in a suitable primary implant seat formed in the mandibular or maxillary bone of the patient. The primary implant seat is previously formed by suitable dental tools, such as cutters or similar. For the purpose of ensuring to fix the main body 2 in the primary implant seat, it preferably comprises an outer thread 3 such to enable to screw the main body 2 in the primary implant seat, which was already threaded. Alternatively, the outer thread 3 can be of the self-tapping type, in order to form the thread in the primary implant seat by screwing the main body 2 itself. The main body 2, so installed and integrated in the patient's bone, forms an artificial root for an artificial tooth to be connected to the main body itself.

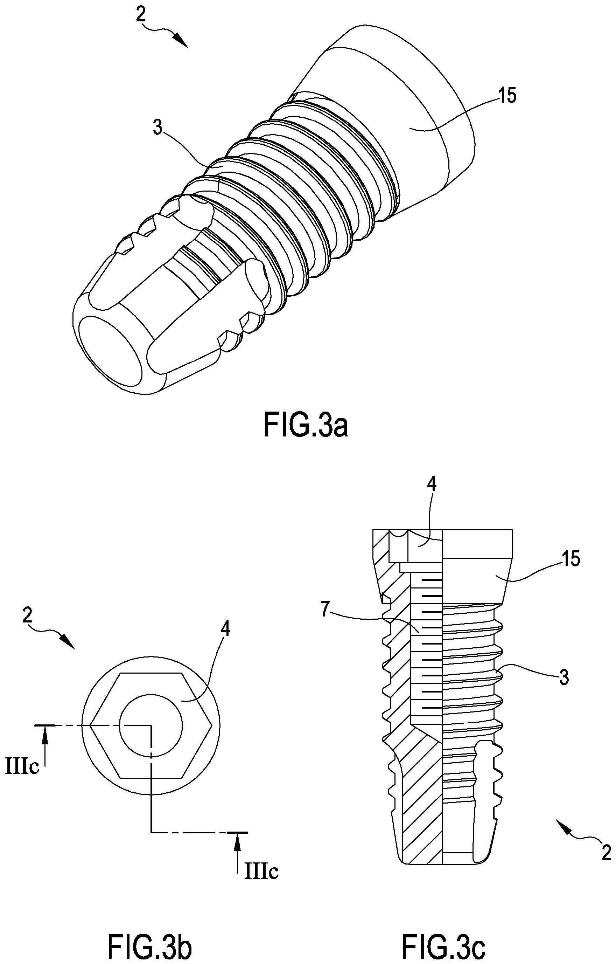

To this purpose, advantageously, the main body 2 comprises an end seat 4 adapted to receive a corresponding end 5 of a rest 6 for the artificial tooth. The end seat 4 of the main body 2 is particularly embodied so that to form a shape coupling with the end 5 of the rest 6 for the artificial tooth. To this purpose, the end seat 4 of the main body 2 and the end 5 of rest 6 for the artificial tooth, have preferably a complementary shape, in order to avoid relative rotations. According to the embodiment shown in Figure 3, the end seat 4 of the main body 2 has an hexagonal shape. Obviously, different shapes of the end seat 4 can be provided in order to form a shape coupling: for example a square, octagonal or generally polygonal shape. Further, the end seat 4 can be of an inner type (according to the illustrated embodiment), in other words a shaped cavity type, or of an outer type, in other words comprising a axially projecting shaped portion (with reference to the longitudinal axis of the main body 2). The rest 6 end 5 for the artificial tooth will be consequently shaped respectively with a shaped projecting portion (according to the illustrated embodiment) or with an inner shaped cavity.

It is to be noted that the main body 2 end seat 4 can be also used for screwing the main body 2 in the primary implant seat. For example, a screwer having an end complementary to the main body 2 end seat 4 can be used.

In order to prevent the rest 6 for the artificial tooth from being axially dislodged from the main body 2, the latter can comprise a preferably inner thread 7, suitable to be coupled to a corresponding screw 8 which can freely slide and rotate in an inner seat of the rest 6 for the artificial tooth. When the screw 8 has been completely screwed, the rest 6 for the artificial tooth is axially blocked with respect to the main body 2 because the head of the screw 8 abuts against a corresponding abutment surface of rest 6.

The rest 6 can be shaped, at its second end 9 opposite to the end 5, in order to enable, by suitable tools, to screw the implant itself in the primary implant seat. For example, with reference to the figures, the end 9 has a squared portion suitable to be operated by suitable spanners. Such squared portion can be thereafter removed, so that the rest 6 takes an arrangement suitable to enable a stable connection of an artificial tooth (not shown in the figures), for example by cementation.

Preferably, the main body 2 is made of titanium or a titanium alloy, of different grades.

It is observed that the main body 2 can have different axial sizes, as a function of the available amount of the cortical bone.

Further, it is observed that, according to an alternative embodiment not illustrated in the figures, the main body 2 and rest 6 can be made in one piece.

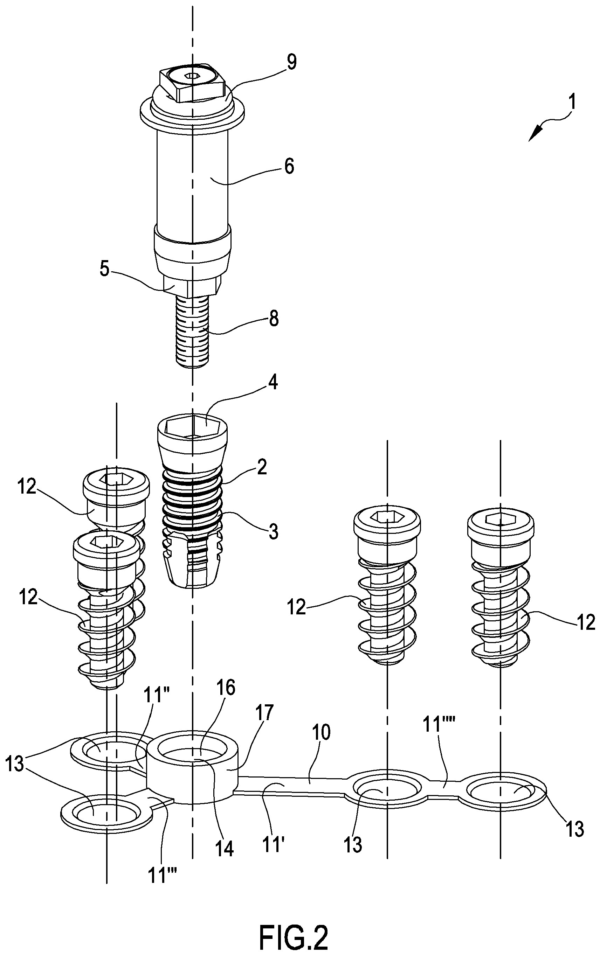

Moreover, the dental implant 1 comprises an auxiliary plate 10 having the function of forming further locking points to the patient's bone in secondary bony fixing zones addition to those in the primary implant seat, where the main body 2 is fixed To this end, the auxiliary plate 10 comprises one or more portions 11 laterally protruding from the main body 2, when the dental implant 1 is assembled. With reference for example to a possible embodiment illustrated in Figure 2, the auxiliary plate 10 comprises a first 11', a second 11" and a third 11'" protruding portions projecting radially outwardly from the main body 2. Further, a fourth protruding portion 11"" aligned with the first protruding portion 11' extends from the first protruding portion 11'. The protruding portions 11, due to their plate shape, are easily foldable by deflection. Therefore, they can be folded in order to be oriented based on the necessities and therefore to be adapted to the shape of the patient's bone. Indeed, the protruding portions 11 are destined to be positioned at the above mentioned secondary bony fixing zones, spaced from the primary implant seat. In this way, if the primary implant seat does not ensure a suitable retainability of the implant, for example due to a scarce amount of bone, particularly of the cortical bone, it is possible to fix the dental implant, by the protruding portions of the auxiliary plate 10, in a plurality of further bony zones, in order to therefore ensure a high retainability of the dental implant once it has been installed.

The dental implant 1 comprises means for fixing the protruding portions 11 of the auxiliary plate at the corresponding auxiliary implant seats formed in the secondary bony fixing zones. The auxiliary implant seats can, if the patient's condition makes it necessary, have a spatial orientation different from the primary implant seat. Particularly, the dental implant comprises fixing screws 12 and the auxiliary plate 10 comprises through holes 13 suitable for inserting the fixing screws 12. Such fixing screws 12 and through holes 13 embody the above mentioned fixing means of the protruding portions 11 of the auxiliary plate in the corresponding auxiliary implant seats. The fixing screws 12 are destined to be inserted in the auxiliary implant seats formed in the patient's bone in positions corresponding to the through holes 13 of the auxiliary plate 10. The abutment of the fixing screws 12 heads in the through holes 13 ensures to block the auxiliary plate 10 to the patient's bone. Under conditions of use, it is not necessary to provide a fixing screw 12 for each through hole 13, but it is sufficient to fix the auxiliary plate by fixing screws in a number less than the total number of the through holes 13 provided in the auxiliary plate 10. The effectively required number of fixing screws 12 depends on the patient's conditions and can be evaluated by the doctor from time to time.

The through holes 13 have a frustoconical trend, as well as the heads of the fixing screws 12, at least at the respective coupling portions, in order to form a rigid conical-type interference fitting between the fixing screws 12 and the auxiliary plate 10. In addition, the through holes 13 can be provided with suitable threads such to enable a threaded connection with the fixing screws 13. This type of coupling causes the fixing screws 12 and auxiliary plate 10 to be integrally connected, in order to form effectively a unitary body, when the implant has been installed.

The fixing screws 12 and auxiliary plate 10 are preferably made of titanium or a titanium alloy, of different grades.

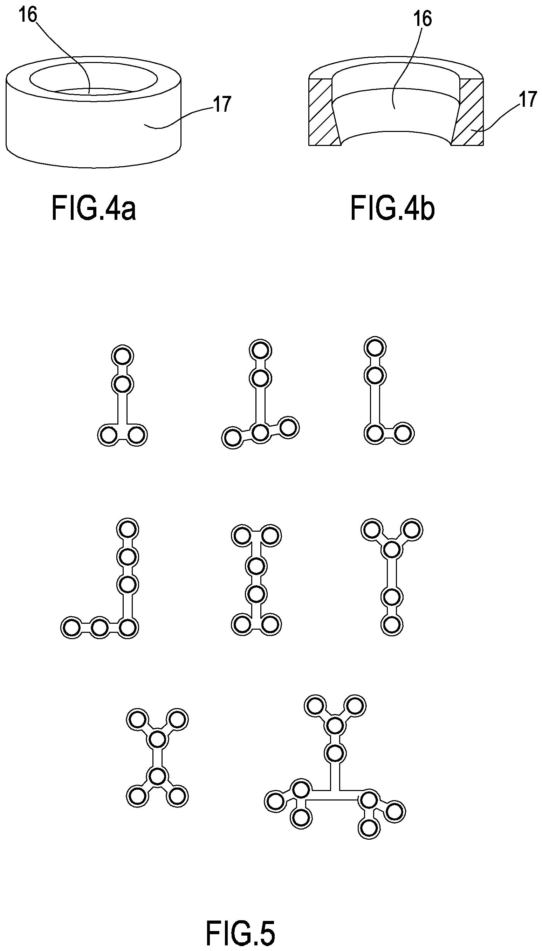

It is observed that the auxiliary plate 10 can take different shapes, according to the specific necessities of the patient. Illustratively, Figure 5 shows some of the several possible shapes of the auxiliary plate 10.

Further, it is observed that the auxiliary plate 10 can have predefined shapes or alternatively can be suitably shaped, for example by rapid prototyping techniques, as a function of the specific shape of the zone where the implant is installed.

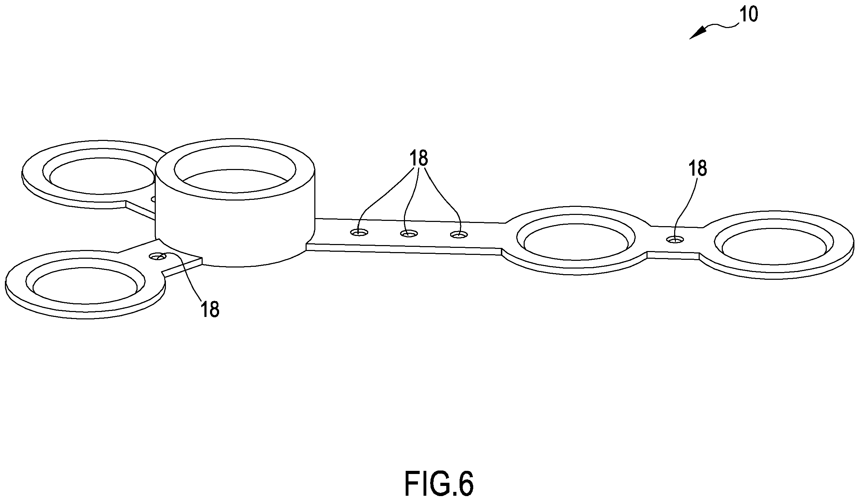

Further, it is to be noted that, according to a possible embodiment (Figure 6), the auxiliary plate 10 can preferably comprise, at the protruding portions 11, a plurality of further holes 18, such to aid the auxiliary plate to be fixed by the spontaneous regeneration of the patient's bone following the implant 1 installation.

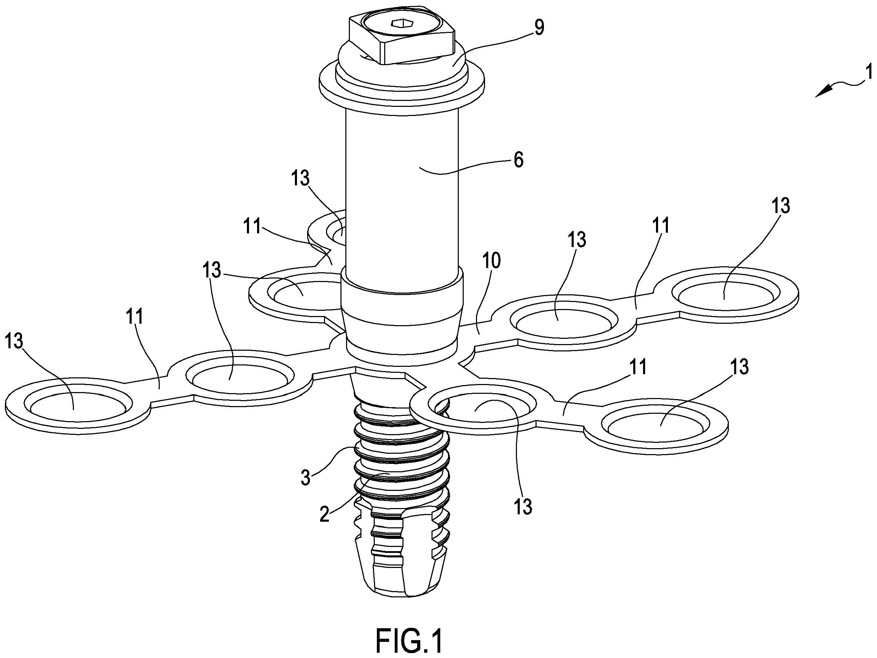

According to a possible embodiment, the main body 2 and auxiliary plate 10 are made in one piece (see for example the embodiment shown in Figure 1).

According to an alternative embodiment, the main body 2 and auxiliary plate 10 are separated and connected to each other, in order to form a unique body only after they have been reciprocally connected (for example, see the embodiment illustrated in Figure 2-4).

According to this latter embodiment, the auxiliary plate 10 comprises a through hole 14 for inserting inside it the main body 2.

In order to obtain the integral connection of the main body 2 to the auxiliary plate 10, the implant 1 can comprise different types of connection means.

According to a possible embodiment, the main body 2 comprises a frustoconical fixing portion 15 (Figures 3a and 3b) and the auxiliary plate 10 comprises a fixing opening 16 also frustoconical (Figure 4b). The fixing opening 16 coincides with or is located at the through hole 14 for inserting the main body 2. The frustoconical shapes of the fixing portion 15 of main body 2 and of fixing opening 15 of main plate 10 make possible an interference rigid fitting of the main body 2 to the auxiliary plate 10 since, in assembled conditions, the main body 2 is wedged in the main plate 10. It is to be observed that such type of frustoconical fitting is the same that can be used for coupling the fixing screws 12 to the through holes 13.

Alternatively, the frustoconical fixing portion 15 and the frustoconical fixing opening 16 can be provided with suitable threads for reciprocally connecting the main body 2 to the main plate 10.

Alternatively, different connecting shapes can be provided. For example, the auxiliary plate 10 and main body 2 can comprise cylindrical connecting portions provided with connecting threads.

Preferably, the fixing opening 16 is formed inside a reinforcing portion 17 of the auxiliary plate 10, made for example by a bushing associated to the auxiliary plate 10 itself. The bushing can be integrally made with the auxiliary plate 10 or can be stably connected to the latter, for example by welding.

Now, a possible process for surgically installing the dental implant according to the invention will be described. It will be made reference to an embodiment wherein the main body and auxiliary plate are separated from each other and connectable, and wherein the rest is separated and the main body 2 is connectable to it.

The process comprises a step of providing the primary implant seat, where the main body 2 will be positioned and fixed.

Such step can comprise the steps of:
- opening the gingival flap;
- forming a lead-in in the cortical bone;
- drilling some passages, for example by cutters of different diameters, in the bone where the main body will be installed in, in order to form the primary implant seat. The cutters can be of mechanical-type or preferably of piezoelectric-type;
- possibly, by a suitable tap chuck, forming a thread in the main implant seat. Such step is not necessary if the thread of the main body is self-tapping.

Further, the process comprises a step of providing the auxiliary implant seats. The latter can be made by following steps substantially analogous to the ones described with reference to the main implant seat.

Further, the process comprises a step of positioning the main plate 10, so that the through hole 14 is in a position coinciding with the main implant seat, and the through holes 13 are in positions coinciding with the auxiliary implant seats. Alternatively, the positioning can be supracrestal or subcrestal. The method can provide a step of folding the protruding portions 11 of the auxiliary plate 10 so that the latter fits on the patient's bone and it is obtained the described alignment of the through holes 13 with the auxiliary implant seats. It is observed that, in case of scarce amount of bone, the protruding portions can be folded in order to keep some lengths thereof at a certain distance from the bone itself, in order to delimit empty spaces which can be used for the bony spontaneous regeneration.

Further, the process comprises a step of inserting the main body 2 in the primary implant seat. In this step, the main body 2 is inserted in the primary implant seat through the through hole 14 of the main plate. When the main body 2 is fixed in the correct position in the main implant seat, the integral connection of the main body to the auxiliary plate is also formed, particularly by coupling the frustoconical fixing portion 15 of the main body 2 to the fixing opening 16 of the auxiliary plate 10.

Moreover, the process comprises a step of inserting and screwing the fixing screws 12 in the auxiliary implant seats through the through holes 13 of the protruding portions 11 of the auxiliary plate 10. It is observed that, for positioning the auxiliary plate, plain compression screws can be used which do not form an integral body with the auxiliary plate, which are then substituted with the fixing screws 12 having the previously described characteristics once the main body has been positioned in the primary implant seat.

The gingival flaps are then closed and required possible sutures are made.

Alternatively, the rest 6 can be connected to the main body 2 immediately following the insertion of the latter in the primary implant seat (the so-called "one stage" technique), or later in time (the so-called "two stage" technique), also after some months, in order to promote a preliminary osteointegration of the main body 2 before applying the rest 6.

Moreover, following the implant fixation, it is possible to position a resorbable membrane such to generate a so-called "curtain effect", in other words such to form a pocket delimiting a space inside which a bony regeneration occurs.

It is to be observed that the dental implant according to the invention can comprise more than one main body 2. For example, two or more main bodies can be provided so that the implant can be fixed in a plurality of points.

Further, more than one implant according to the invention, for example connected to each other by auxiliary plates exploiting the through holes 13 of the protruding portions 11 of the auxiliary plate 10 for fixing them, can be implanted in the patient.

It is to be observed that the dental implant according to the invention can be used as zygomatic implant. In such case, the main body 2 can have large axial dimensions so that it can be inserted in a position such it extends to the zygomatic bone or in proximity with the same. However, the main body 2 can be maintained within sizes suitable to avoid the risk of accidentally entering the orbital area of the patient.

According to a further variant, an antral window is formed, preferably by a piezoelectric instrumentations, at the primary implant seat, wherein the portion of the auxiliary plate 10 having the fixing opening 16 is inserted into, which is engaged to the main body when this is inserted in the primary implant seat.

Further, it is to be noted that the dental implant according to the invention can be exploited for the bony regeneration. Particularly, the auxiliary plate can be positioned so that it is raised in zones devoid of bone, in order to leave empty a space. The empty space can be filled by a biomaterial and all is covered by a resorbable membrane. This promotes the bony regeneration in the above cited space.

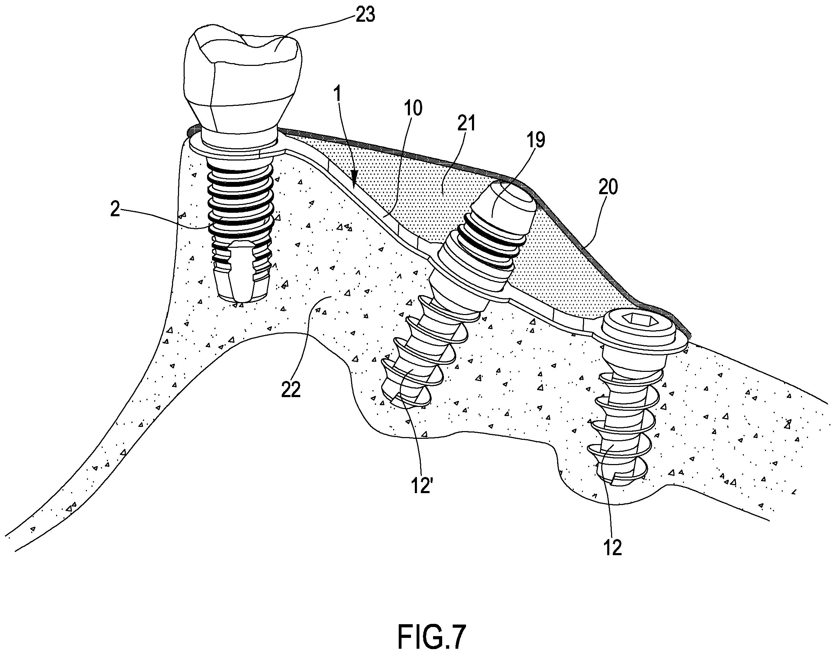

According to a further alternative embodiment, one or more fixing screws 12 (indicated by 12') comprise an auxiliary portion 19 adapted to protrude outside the secondary implant seat. Such auxiliary portion 19 can have different functions. With reference to the embodiment shown in Figure 7, the screw 12' has an auxiliary portion 19 acting, in this case, as a spacing element for positioning a resorbable membrane 20. The space 21 delimited between the membrane 20 and bone 21 of the patient, promotes the bony regeneration. In this way, once the regeneration is completed, the auxiliary plate 10 is entirely osteointegrated. Figure 7 shows also an artificial tooth 22 associated to the implant 1 main body 2.

Alternatively, the auxiliary portion 19 can have different functions. For example, it can act as a connecting element of the dental implant 1 with orthodontic appliances, orthodontic braces, osteodistractors, or similar. In this case, the auxiliary portion can be caused to emerge at least partially from the patient's gingiva.

The auxiliary portion 19 can be smooth or threaded according to its use. It preferably comprises lead-ins which make easier to screw the screws 12' in the secondary implant seat. Preferably, the auxiliary portion 19 is made in titanium or a titanium alloy.

From the above given description, the person skilled in the art can appreciate how the dental implant according to the invention ensures an effective retainability also if the primary implant seat is characterized by a scarce bone amount, particularly of the cortical bone. Indeed, the presence of the auxiliary plate enables to fix the implant also in zones more favorable with reference to presence of more bone amount, particularly of the cortical type, by ensuring therefore a suitable retainability of the same.

Moreover, the coupling modes between the main body, auxiliary plate and fixing screws of the auxiliary plate, make possible that the implant, once installed, forms a rigid integral body with the osteointegrated surface.

Further, the procedure of installing the implant is not necessarily complex and is not necessarily invasive for the patient in comparison with different so-called techniques used in case of shortage of cortical bone. The risk of errors on the part of the physicist and of complications for the patient is therefore lower.

Lastly, the dental implant, according to the invention, due to its deformability of the protruding portions of the auxiliary plate, is versatile and adaptable to the shape of a single patient and to other specific problems of each case.

To the described embodiments of the dental implant according to the invention, a person skilled in the art in order to meet specific contingent needs, can introduce several additions, modifications or substitutions of elements with other operatively equivalent, without however departing from the scope of the attached claims.

## Claims

1. Dental implant (1) comprising:
- a main body (2) suitable for being implanted in a primary implant seat formed in a mandibular or maxillary bone of a patient, so to form an artificial root for an artificial tooth;
- an auxiliary plate (10) comprising one or more protruding portions (11) laterally protruding relative to the main body (2) and foldable by deflection, so that they can be positioned at secondary bony fixing zones at a distance from the primary implant seat when the main body (2) is implanted in the primary implant seat;
- screws (12) for fixing the one or more protruding portions (11), in corresponding one or more auxiliary implant seats formed in the secondary bony fixing zones,
wherein:
said main body (2) and said auxiliary plate (10) are integrally formed in one-piece or are separated and integrally connectable to each other;
said one or more protruding portions (11) of the auxiliary plate (10) comprise respective second through holes (13) for fixing by means of fixing screws (12) the auxiliary plate (10) in the one or more auxiliary implant seats formed in the secondary bony fixing zones, said second through holes (13) and the heads of said fixing screws (12) having a frustoconical trend, such to form a conical-type, rigid, interference fitting between the fixing screws (12) and the auxiliary plate (10).

2. Dental implant (1) according to claim 1, wherein said main body (2) and said auxiliary plate (10) are separated and integrally connectable to each other and said auxiliary plate (10) comprises at least one first through hole (14) for inserting the main body (2) in the primary implant seat through the first through hole (14) itself.

3. Dental implant (1) according to claim 2, wherein said main body (2) comprises a frustoconical fixing portion (15) and said auxiliary plate (10) comprises a frustoconical fixing opening (16) which is coincident or is arranged at said first through hole (14) for interference fitting said main body (2) to said auxiliary plate (10).

4. Dental implant (1) according to claim 2, wherein said main body (2) comprises a threaded frustoconical or cylindrical fixing portion (15) and said auxiliary plate (10) comprises a threaded frustoconical or cylindrical fixing opening which is coincident or is arranged at said first through hole (14) of the auxiliary plate (10) for connecting said main body (2) to said auxiliary plate (10) by screwing the main body (2) fixing portion (15) into the auxiliary plate (10) fixing opening (16).

5. Dental implant (1) according to claim 3 or 4, wherein said auxiliary plate (10) fixing opening (16) is formed inside a reinforcing portion (17) of the auxiliary plate (10).

6. Dental implant (1) according to anyone of the preceding claims, wherein said main body (2) comprises an external thread (3) for screwing the main body (2) itself into the primary implant seat.

7. Dental implant (1) according to anyone of claims 1-6, comprising a rest (6) for the artificial tooth which is connectable to the main body (2), wherein said main body (2) comprises an end seat (4) suitable to house a corresponding end (5) of said rest (6) for the artificial tooth.

8. Dental implant (1) according to anyone of claims 1-6, comprising a rest (6) for the artificial tooth which is made in one piece with said main body (2).

9. Dental implant (1) according to anyone of the preceding claims, wherein said auxiliary plate (10) comprises a plurality of further holes (18), such to aid in fixing the auxiliary plate (10) itself as a consequence of the regeneration of the patient's bone.

10. Dental implant (1) according to anyone of the preceding claims, wherein one or more (12') of the fixing screws (12) comprise an auxiliary portion (19) adapted to protrude outside the secondary implant seat.

11. Dental implant (1) according to claim 10, wherein said auxiliary portion (19) forms a spacing element for positioning a resorbable membrane (20), such to delimit a space (21) for aiding the bony regeneration.

12. Dental implant (1) according to claim 10, wherein said auxiliary portion (19) forms an element for connecting the dental implant (1) to the orthodontic appliances, orthodontic braces, osteodistractors, or similar.

## Patentansprüche

1. Zahnimplantat (1) umfassend:
- einen Hauptkörper (2), der geeignet ist, in einen primären Implantatsitz implantiert zu werden, der in einem Unterkiefer- oder Oberkieferknochen eines Patienten ausgebildet ist, um so eine künstliche Wurzel für einen künstlichen Zahn zu bilden;
- eine Hilfsplatte (10), die einen oder mehrere vorstehende Abschnitte (11) umfasst, die in Bezug auf den Hauptkörper (2) seitlich vorstehen und durch Ablenkung faltbar sind, so dass sie in sekundären knöchernen Fixierungszonen in einem Abstand vom primären Implantatsitz positioniert werden können, wenn der Hauptkörper (2) in den primären Implantatsitz eingesetzt wird;
- Schrauben (12) zum Befestigen des einen oder mehrerer vorstehender Abschnitte (11) in entsprechenden einem oder mehreren zusätzlichen Implantatsitzen, die in den sekundären knöchernen Befestigungszonen ausgebildet sind,
wobei:
der Hauptkörper (2) und die Hilfsplatte (10) integral einteilig ausgebildet sind oder getrennt und integral miteinander verbindbar sind;
der eine oder die mehreren vorstehenden Abschnitte (11) der Hilfsplatte (10) jeweils zweite Durchgangsbohrungen (13) zur Befestigung der Hilfsplatte (10) mittels Befestigungsschrauben (12) in dem einen oder den mehreren Hilfsimplantatsitzen umfassen, die in den sekundären knöchernen Befestigungszonen ausgebildet sind, wobei die zweiten Durchgangslöcher (13) und die Köpfe der Befestigungsschrauben (12) einen kegelstumpfförmigen Trend aufweisen, um eine konische, starre Interferenzpassung zwischen den Befestigungsschrauben (12) und der Hilfsplatte (10) zu bilden.

2. Zahnimplantat (1) nach Anspruch 1, wobei der Hauptkörper (2) und die Hilfsplatte (10) getrennt und integral miteinander verbindbar sind und die Hilfsplatte (10) mindestens ein erstes Durchgangsloch (14) zum Einsetzen des Hauptkörpers (2) in den primären Implantatsitz durch das erste Durchgangsloch (14) selbst aufweist.

3. Zahnimplantat (1) nach Anspruch 2, wobei der Hauptkörper (2) einen kegelstumpfförmigen Befestigungsabschnitt (15) umfasst und die Hilfsplatte (10) eine kegelstumpfförmige Befestigungsöffnung (16) umfasst, die übereinstimmend ist oder an der ersten Durchgangsbohrung (14) angeordnet ist, um den Hauptkörper (2) mit der Hilfsplatte (10) in Eingriff zu bringen.

4. Zahnimplantat (1) nach Anspruch 2, wobei der Hauptkörper (2) einen kegelstumpfförmigen oder zylindrischen Befestigungsabschnitt (15) mit Gewinde umfasst und die Hilfsplatte (10) eine kegelstumpfförmige oder zylindrische Befestigungsöffnung mit Gewinde umfasst, die übereinstimmend ist oder an der ersten Durchgangsbohrung (14) der Hilfsplatte (10) angeordnet ist, um den Hauptkörper (2) mit der Hilfsplatte (10) zu verbinden, indem der Hauptkörper (2) in die Befestigungsöffnung (16) der Hilfsplatte (10) eingeschraubt wird.

5. Zahnimplantat (1) nach Anspruch 3 oder 4, wobei die Befestigungsöffnung (16) der Zusatzplatte (10) innerhalb eines Verstärkungsabschnitts (17) der Zusatzplatte (10) ausgebildet ist.

6. Zahnimplantat (1) nach einem der vorstehenden Ansprüche, wobei der Hauptkörper (2) ein Außengewinde (3) zum Verschrauben des Hauptkörpers (2) selbst in den primären Implantatsitz umfasst.

7. Zahnimplantat (1) nach einem der Ansprüche 1 bis 6, umfassend eine Auflage (6) für den künstlichen Zahn, die mit dem Hauptkörper (2) verbindbar ist, wobei der Hauptkörper (2) einen Endsitz (4) umfasst, der geeignet ist, ein entsprechendes Ende (5) der Auflage (6) für den künstlichen Zahn aufzunehmen.

8. Zahnimplantat (1) nach einem der Ansprüche 1 bis 6, umfassend eine Auflage (6) für den künstlichen Zahn, die in einem Stück mit dem Hauptkörper (2) hergestellt ist.

9. Zahnimplantat (1) nach einem der vorstehenden Ansprüche, wobei die Zusatzplatte (10) mehrere weitere Löcher (18) umfasst, um die Befestigung der Zusatzplatte (10) selbst als Folge der Regeneration des Knochens des Patienten zu erleichtern.

10. Zahnimplantat (1) nach einem der vorstehenden Ansprüche, wobei eine oder mehrere (12') der Befestigungsschrauben (12) einen Hilfsabschnitt (19) umfassen, der so angepasst ist, dass er aus dem sekundären Implantatsitz herausragt.

11. Zahnimplantat (1) nach Anspruch 10, wobei der Hilfsabschnitt (19) ein Abstandelement zum Positionieren einer resorbierbaren Membran (20) bildet, um einen Raum (21) zur Unterstützung der knöchernen Regeneration abzugrenzen.

12. Zahnimplantat (1) nach Anspruch 10, wobei der Hilfsabschnitt (19) ein Element zum Verbinden des Zahnimplantats (1) mit den kieferorthopädischen Geräten, kieferorthopädischen Zahnspangen, Osteodistraktoren oder dergleichen bildet.

## Revendications

1. Implant dentaire (1) comprenant :
- un corps principal (2) approprié pour être implanté dans une assise d'implant primaire formée dans un os mandibulaire ou maxillaire d'un patient, de façon à former une racine artificielle pour une dent artificielle ;
- une plaque auxiliaire (10) comprenant une ou plusieurs parties saillante (11) dépassant latéralement par rapport au corps principal (2) et pliables par déflexion, de sorte qu'elles peuvent être positionnées au niveau de zones de fixation osseuse secondaires à une certaine distance de l'assise d'implant primaire quand le corps principal (2) est implanté dans l'assise d'implant primaire ;
- des vis (12) destinées à fixer la ou les parties saillantes (11) en faisant correspondre une ou plusieurs assises d'implant auxiliaires formées dans les zones de fixation osseuse secondaires,
où :
ledit corps principal (2) et ladite plaque auxiliaire (10) sont intégralement formés d'une pièce ou sont séparés et intégralement raccordables l'un à l'autre ;
lesdites une ou plusieurs parties saillantes (11) de la plaque auxiliaire (10) comprennent des seconds orifices traversants respectifs (13) pour fixer au moyen de vis de fixation (12) la plaque auxiliaire (10) dans l'une ou plusieurs assises d'implant auxiliaires formées dans les zones de fixation osseuse secondaires, lesdits seconds orifices traversants (13) et les têtes desdites vis de fixation (12) ayant une tendance frustoconique de façon à former un ajustement de type conique, rigide, avec serrage entre les vis de fixation (12) et la plaque auxiliaire (10).

2. Implant dentaire (1) selon la revendication 1, dans lequel ledit corps principal (2) et ladite plaque auxiliaire (10) sont séparés et intégralement raccordables l'un à l'autre et ladite plaque auxiliaire (10) comprend au moins un premier orifice traversant (14) pour insérer le corps principal (2) dans l'assise d'implant primaire à travers le premier orifice traversant (14) lui-même.

3. Implant dentaire (1) selon la revendication 2, dans lequel ledit corps principal (2) comprend une partie de fixation frustoconique (15) et ladite plaque auxiliaire (10) comprend une ouverture de fixation frustoconique (16) qui coïncide ou est disposée au niveau dudit premier orifice traversant (14) pour l'ajustement avec serrage dudit corps principal (2) à ladite plaque auxiliaire (10).

4. Implant dentaire (1) selon la revendication 2, dans lequel ledit corps principal (2) comprend une partie de fixation frustoconique ou cylindrique (15) et ladite plaque auxiliaire (10) comprend une ouverture de fixation frustoconique ou cylindrique filetée qui coïncide ou est disposée au niveau dudit premier orifice traversant (14) de la plaque auxiliaire (10) pour raccorder ledit corps principal (2) à ladite plaque auxiliaire (10) en vissant la partie de fixation (15) du corps principal (2) dans l'ouverture de fixation (16) de la plaque auxiliaire (10).

5. Implant dentaire (1) selon la revendication 3 ou 4, dans lequel ladite ouverture de fixation (16) de la plaque auxiliaire (10) est formée à l'intérieur d'une partie de renfort (17) de la plaque auxiliaire (10).

6. Implant dentaire (1) selon l'une quelconque des revendications précédentes, dans lequel ledit corps principal (2) comprend un filetage externe (3) pour visser le corps principal (2) lui-même dans l'assise d'implant primaire.

7. Implant dentaire (1) selon l'une quelconque des revendications 1 à 6, comprenant un taquet (6) pour la dent artificielle qui est raccordable au corps principal (2), ledit corps principal (2) comprenant une assise terminale (4) appropriée pour accueillir une extrémité correspondante (5) dudit taquet (6) pour la dent artificielle.

8. Implant dentaire (1) selon l'une quelconque des revendications 1 à 6, comprenant un taquet (6) pour la dent artificielle qui est constitué d'une seule pièce avec ledit corps principal (2).

9. Implant dentaire (1) selon l'une quelconque des revendications précédentes, dans lequel ladite plaque auxiliaire (10) comprend une pluralité d'autres orifices (18) de façon à faciliter la fixation de la plaque auxiliaire (10) elle-même par suite de la régénération de l'os du patient.

10. Implant dentaire (1) selon l'une quelconque des revendications précédentes, dans lequel une ou plusieurs (12') des vis de fixation (12) comporte une partie auxiliaire (19) appropriée pour dépasser en dehors de l'assise d'implant secondaire.

11. Implant dentaire (1) selon la revendication 10, dans lequel ladite partie auxiliaire (19) forme un élément d'espacement pour positionner une membrane résorbable (20) de façon à délimiter un espace (21) pour faciliter la régénération osseuse.

12. Implant dentaire (1) selon la revendication 10, dans lequel ladite partie auxiliaire (19) forme un élément pour raccorder l'implant dentaire (1) aux appareils orthodontiques, aux appareils dentaires, aux ostéo-distracteurs ou à des appareils similaires.
